(19) Europäisches Patentamt
European Patent Office
Office européen des brevets

(11) Publication number: **0 048 501**
**B1**

(12) # EUROPEAN PATENT SPECIFICATION

(45) Date of publication of patent specification: **27.07.83**

(21) Application number: **81108507.5**

(22) Date of filing: **02.11.79**

(60) Publication number of the earlier application in accordance with Art. 76 EPC: **0011417**

(51) Int. Cl.³: **C 07 D 487/04**
//(C07D487/04, 235/00, 209/00)

(54) **Optically active or racemic diazabicyclooctane derivatives.**

(30) Priority: **02.11.78 JP 135511/78
06.11.78 JP 137147/78
04.06.79 JP 70365/79
04.06.79 JP 70366/79
04.06.79 JP 70367/79**

(43) Date of publication of application:
**31.03.82 Bulletin 82/13**

(45) Publication of the grant of the patent:
**27.07.83 Bulletin 83/30**

(84) Designated Contracting States:
**DE FR GB IT NL**

(56) References cited:
**CHEMISTRY LETTERS, No. 6, 1979 T. MUKAIYAMA et al. "Versatile Method for the Preparation of Optically Active alpha-hydroxy Aldehydes with Desired Configurations" pages 705 to 708
CHEMISTRY LETTERS, No. 8, 1979 Y. SAKITO et al. "Asymmetric Synthesis of Two Enantiomers of Frontalin" pages 1027 to 1028**

(73) Proprietor: **SUMITOMO CHEMICAL COMPANY, LIMITED
15 Kitahama 5-chome Higashi-ku
Osaka-shi Osaka-fu (JP)**

(72) Inventor: **Mukaiyama, Teruaki
15-18 Minamiogikubo-1-chome
Suginami-ku Tokyo (JP)**
Inventor: **Sakito, Yoji
1-27 Kitamachi-4-chome
Kichijoji Musashino-shi (JP)**
Inventor: **Asami, Masatoshi
1272-27 Kudencho
Totsuka-ku Yokohama (JP)**

(74) Representative: **Overin, Alison Diana et al,
J.A. KEMP & CO. 14, South Square Gray's Inn
London WC1R 5EU (GB)**

(56) References cited:
**CHEMISTRY LETTERS, No. 11, 1978 T. MUKAIYAMA et al. "Asymmetric Synthesis of alpha-hydroxy Aldehydes" pages 1253 to 1256**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 048 501 B1**

Optically active or racemic diazabicyclooctane derivatives

This invention relates to optically active or racemic diazabicyclooctane derivatives and a process for preparing them.

According to this invention, there is provided an optically active or racemic diazabicyclooctane derivative of formula

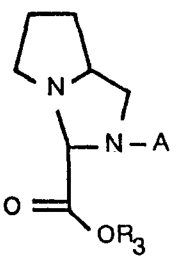

(4)

wherein A represents a $C_6$—$C_{14}$ aryl group or a $C_1$—$C_4$ alkyl or alkoxy group- or halogen-substituted $C_6$—$C_{14}$ aryl group and $R_3$ represents a $C_1$—$C_4$ alkyl group.

The diazabicyclooctane derivatives of this invention can be obtained by allowing a glyoxylate ester or a glyoxylate ester hemiacetal to react with an optically active or racemic 2-(N-substituted aminomethyl)pyrrolidine of formula

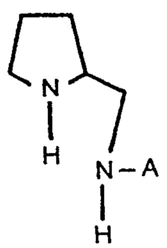

(3)

wherein A is as defined above.

The compounds of this invention are useful intermediates in the synthesis of $\alpha$-hydroxyaldehydes of formula

$$R_1\text{—}\overset{\overset{\large CHO}{|}}{\underset{\underset{\large R_2}{|}}{C}}\text{—}OH \qquad (2)$$

wherein $R_1$ represents a $C_6$—$C_{14}$ aryl group, $C_1$—$C_{10}$ alkyl group, cyclohexyl, $C_3$—$C_{10}$ alkenyl group, cyclohexenyl, $C_2$—$C_{10}$ alkynyl group, $C_7$—$C_{14}$ aralkyl group or one of these groups substituted by a $C_1$—$C_4$ alkoxy group and $R_2$ represents a $C_1$—$C_{10}$ alkyl group (preferably a $C_2$—$C_{10}$ alkyl group), cyclohexyl, $C_2$—$C_{10}$ alkenyl group, cyclohexenyl, $C_2$—$C_{10}$ alkynyl group, $C_7$—$C_{14}$ aralkyl group, $C_6$—$C_{14}$ aryl group, or one of these groups substituted by a $C_1$—$C_4$ alkoxy group or an ether thereof, which are themselves useful intermediates for the preparation of pharmaceuticals and agricultural chemicals. Particularly, the optically active $\alpha$-hydroxyaldehydes are of great significance. For instance, atrolactamide derivable from 2-hydroxy-2-phenylpropion-aldehyde is used as a pharmaceutical. These intermediates, furthermore, are used also in synthesizing frontalin and derivatives thereof which are aggregation pheromones.

In preparing the $\alpha$-hydroxyaldehydes of formula (2), the compounds of formula (4) of the present invention may be converted to an optically active or racemic aminal of formula

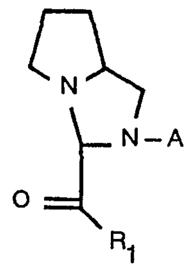

(1)

wherein A and $R_1$ are as defined above, by reaction with a Grignard reagent of formula $R_1MgX$, wherein $R_1$ is as defined above and X is a halogen atom such as Cl, Br or I. The compound of formula (1) so produced may then be reacted to form an $\alpha$-hydroxyaldehyde of formula (2) by reaction with a Grignard reagent of formula $R_2MgX$ wherein $R_2$ and X are as identified above, and subsequently hydrolysing the reaction product. These reaction processes leading from the compound of the present invention to the $\alpha$-hydroxyaldehydes of formula (2) (and the formula (1) compounds) form the subject of EP—A—0011417 from which this Application is divided. Most preferably, optically active or racemic $\alpha$-hydroxyaldehydes are obtained when $R_1$ and $R_2$ are different from each other. These optically active $\alpha$-hydroxyaldehydes and certain of the racemic $\alpha$-hydroxyaldehydes, and benzyl ethers thereof form the subject of EP—A—0050351, which is also divided from EP—A—0011417.

The invention is described below in detail.

Glyoxylate esters or glyoxylate ester hemiacetals used in preparing the diazabicyclooctane derivatives of this invention are generally $C_1$—$C_4$ alkyl esters such as methyl glyoxylate, ethyl glyoxylate, n-propyl glyoxylate, isopropyl glyoxylate, n-butyl glyoxylate, methyl hydroxymethoxyacetate, ethyl hydroxyethoxyacetate, n-propyl hydroxy-n-propoxyacetate, isopropyl hydroxyisopropoxyacetate, n-butyl hydroxy-n-butoxyacetate and the like. The alkyl groups of these esters correspond to $R_3$ in formula (4).

2-(N-substituted aminomethyl)pyrrolidines can be prepared easily, for example, from proline [Bull. Chem. Soc., Japan, *51*, 1869 (1978)]. The most preferably used are those of formula (3) in which A is a $C_6$—$C_{14}$ aryl group or a $C_1$—$C_4$ alkoxy- or halogen-substituted

$C_6$—$C_{14}$ aryl group, such as 2 - (anilinomethyl)pyrrolidine, 2 - (2,6 - xylidinomethyl)-pyrrolidine, 2 - (N - p - tolylaminomethyl)-pyrrolidine and 2 - (N - naphthylaminomethyl)pyrrolidine. Particular examples for A of the formula (4) are aryl groups such as phenyl, 2,6-xylyl, p-tolyl and naphthyl. These groups may also be substituted with a halogen or $C_1$—$C_4$ alkoxy group.

The reaction between optically active or racemic 2 - (N - substituted aminomethyl)-pyrrolidines and glyoxylate esters or glyoxylate ester hemiacetals to prepare the compounds of the present invention is carried out generally in the presence of a common organic solvent such as benzene, toluene, ether, chloroform, hexane, heptane or the like. Although the reaction temperature is subject to no particular limitation, it is generally in the range of −30° to 200°C and preferably below the boiling point of the solvent employed. If the reaction is carried out at a temperature higher than the boiling point of the solvent, the reaction system must be kept under a superatmospheric pressure in a closed vessel. The water formed with the progress of reaction should be removed by the use of a dehydrating agent such as molecular sieve or by azeotropic distillation. The removal of water by azeotropic distillation with benzene or toluene is convenient and economical. The diazabicyclooctane derivative of the formula (4) thus obtained can be purified by column chromatography or distillation, but a crude product obtained after removal of the solvent by distillation can be used without further purification. Examples of diazabicyclooctane derivatives obtainable in the above manner include 2 - alkoxycarbonyl - 3 - phenyl - 1, 3 - diazabicyclo[3,3,0]octanes such as 2 - methoxycarbonyl - 3 - phenyl - 1, 3 - diazabicyclo[3,3,0]octane, 2 - ethoxycarbonyl - 3 - phenyl - 1,3 - diazabicyclo[3,3,0]octane and 2 - butoxycarbonyl - 3 - phenyl - 1,3 - diazabicyclo[3,3,0]octane and the like. It will be easily understood that other diazabicyclooctane derivatives can also be obtained in a similar manner.

The invention is illustrated below in detail with reference to Examples.

Example 1

In 10 ml of benzene, were dissolved 683 mg of methyl hydroxymethoxyacetate and 1.00 g of (S)-2-(anilinomethyl)pyrrolidine. The resulting solution was refluxed for 30 minutes while removing the water by azeotropic distillation. On removing the solvent by distillation in vacuo, there were obtained 1.38 g of 2 - methoxycarbonyl - 3 - phenyl - 1,3 - diazabicyclo[3,3,0]octane, a derivative of glyoxylic acid. NMR peaks: δ (ppm) = 1.5—2.3 (4H, multiplet), 2.3—4.1 (5H, multiplet), 3.5 (3H, singlet), 4.6 (1H, singlet), 6.2—7.1 (5H, multiplet). After having been purified by alumina column chromatography and short-path distillation, the substance showed the following results of elementary analysis:

| | C% | H% | N% |
|---|---|---|---|
| Found | 68.26 | 7.64 | 11.65 |
| Calculated | 68.27 | 7.37 | 11.37 |

Example 2

Except that 500 mg of methyl glyoxylate was used in place of methyl hydroxymethoxyacetate, the procedure of Example 1 was repeated to obtain 1.38 g of 2-methoxycarbonyl - 3 - phenyl - 1,3 - diazabicyclo[3,3,0]octane.

Example 3

Except that 842 mg of ethyl hydroxyethoxyacetate was used in place of methyl hydroxymethoxyacetate, the procedure of Example 1 was followed to obtain 1.44 g of 2 - ethoxycarbonyl - 3 - phenyl - 1,3 - diazabicyclo-[3,3,0]octane.
NMR peaks: δ (ppm) = 1.2 (3H, triplet), 1.5—2.3 (4H, multiplet), 2.3—4.1 (5H, multiplet), 4.0 (2H, quartet), 4.6 (1H, singlet), 6.2—7.1 (5H, multiplet).

Example 4

Except that 740 mg of n-butyl glyoxylate was used in place of methyl hydroxymethoxyacetate, the procedure of Example 1 was followed to obtain 1.60 g of 2 - butoxycarbonyl - 3 - phenyl - 1,3 - diazabicyclo-[3,3,0]octane.
NMR peaks: δ (ppm) = 0.7—2.3 (11H, multiplet), 2.3—4.1 (5H, multiplet), 3.9 (2H, triplet), 4.6 (1H, singlet), 6.2—7.1 (5H, multiplet).

**Claims**

1. An optically active or racemic diazabicyclooctane derivative of formula

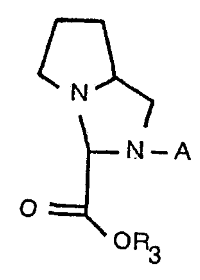

wherein A represents a $C_6$—$C_{14}$ aryl group or a $C_1$—$C_4$ alkyl or alkoxy group- or halogen-substituted $C_6$—$C_{14}$ aryl group and $R_3$ represents a $C_1$—$C_4$ alkyl group.

2. An optically active or racemic diazabicyclooctane derivative of formula

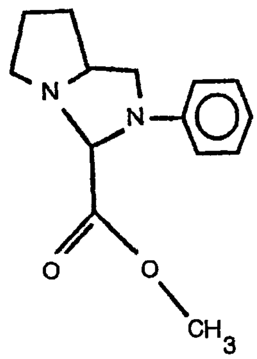

3. An optically active or racemic diazabicyclooctane derivative of formula

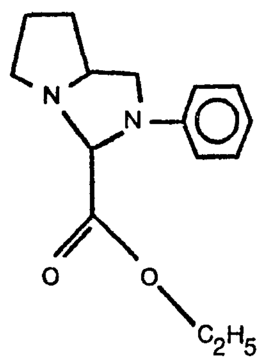

4. An optically active or racemic diazabicyclooctane derivative of formula

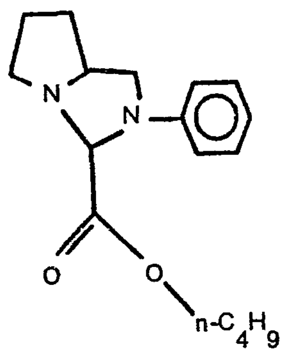

5. A process for preparing an optically active or racemic diazabicyclooctane derivative of formula

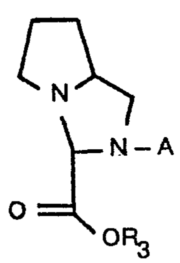

wherein A and $R_3$ are as defined in any one of the preceding claims, which comprises reacting a $C_{1-4}$ alkyl glyoxylate or a $C_{1-4}$ alkyl glyoxylate hemiacetal with an optically active or racemic 2-(N-substituted aminomethyl) pyrrolidine of formula

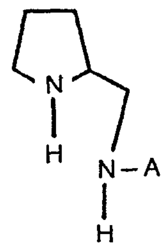

wherein A is as defined in any one of the preceding claims.

**Revendications**

1. Dérivé énantiomère ou racémique de diazabicyclooctane de formule

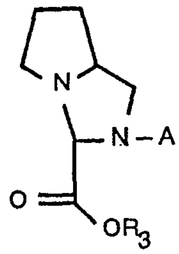

dans laquelle A représente un groupe aryle en C6—C14 ou un groupe aryle en C6—C14 substitué par des groupes alkyle ou alcoxy en C1—C4 ou des halogènes et $R_3$ représente un groupe alkyle en C1—C4.

2. Dérivé énantiomère ou racémique du diazabicyclooctane de formule

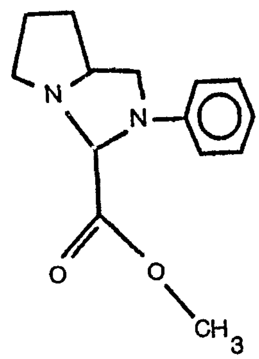

3. Dérivé énantiomère ou racémique du diazabicyclooctane de formule

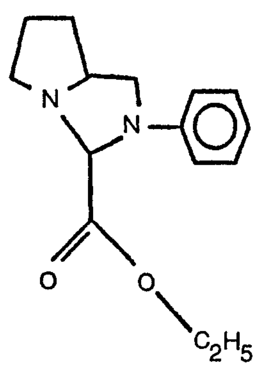

4. Dérivé énantiomère ou racémique du diazabicyclooctane de formule

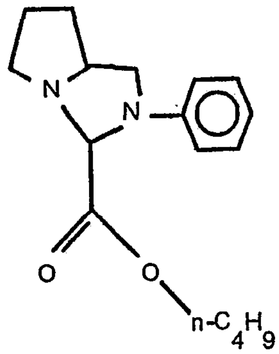

5. Procédé pour préparer un dérivé énantiomère ou racémique du diazabicyclooctane de formule

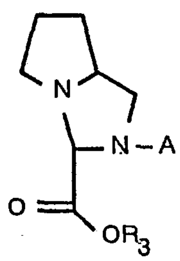

dans laquelle A et $R_3$ sont tels que définis dans l'une quelconque des revendications qui précèdent, procédé qui consiste à faire réagir un glyoxylate d'alkyle en C1—C4 ou un hémiacétal d'un glyoxylate d'alkyle en C1—C4 avec une 2-(aminométhyle N-substitué)-pyrrolidine énantiomère ou racémique de formule

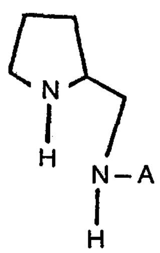

dans laquelle A est tel que défini dans l'une quelconque des revendications qui précèdent.

**Patentansprüche**

1. Optisch aktives oder racemisches Diazabicyclooctanderivat der Formel

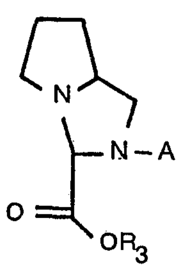

worin bedeuten:
A eine $C_6$—$C_{14}$-Arylgruppe oder eine $C_1$—$C_4$-alkyl- oder -alkoxy- oder halogensubstituierte $C_6$—$C_{14}$- Arylgruppe und
$R_3$ eine $C_1$—$C_4$-Alkylgruppe.

2. Optisch aktives oder racemisches Diazabicyclooctanderivat der Formel:

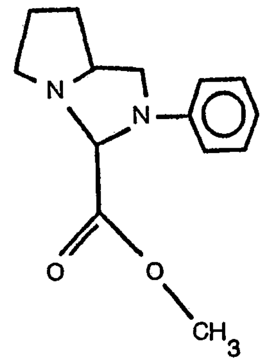

3. Optisch aktives oder racemisches Diazabicyclooctanderivat der Formel:

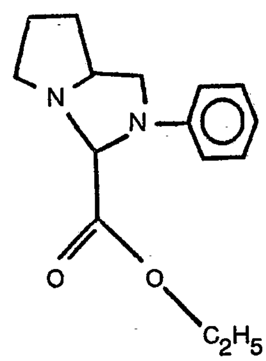

4. Optisch aktives oder racemisches Diazabicyclooctanderivat der Formel:

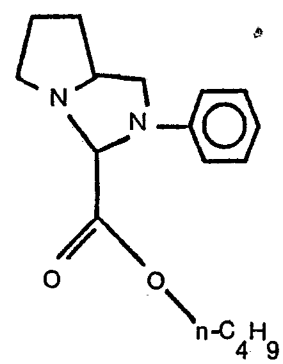

5. Verfahren zur Herstellung eines optisch aktiven oder racemischen Diazabicyclooctanderivats der Formel:

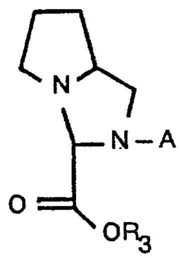

worin A und $R_3$ die angegebene Bedeutung besitzen, dadurch gekennzeichnet, daß man ein $C_1$—$C_4$-Alkylglyoxylat oder ein $C_1$—$C_4$-Alkylglyoxylat-hemiacetal mit einem optisch aktiven oder racemischen 2-(N-substituierten Aminomethyl)pyrrolidin der Formel:

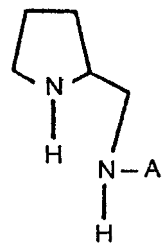

worin A die angegebene Bedeutung besitzt, umsetzt.